# EUROPÄISCHE PATENTSCHRIFT

(11) **EP 0 685 088 B1**
(45) Veröffentlichungstag und Bekanntmachung des Hinweises auf die Patenterteilung: **13.09.2000**
(21) Anmeldenummer: 94906873.8
(22) Anmeldetag: 15.02.1994
(51) Int. Cl.: G06F 17/00

(54) **VERFAHREN UND VORRICHTUNG ZUR PLANUNG UND KONTROLLE EINES CHIRURGISCHEN EINGRIFFS**
PROCESS AND DEVICE FOR PLANNING AND MONITORING A SURGICAL OPERATION
PROCEDE ET APPAREIL PERMETTANT DE PROGRAMMER ET DE CONTROLER LE DEROULEMENT D'UNE INTERVENTION CHIRURGICALE

(30) Priorität: 16.02.1993 DE 4304571
(43) Veröffentlichungstag der Anmeldung: 06.12.1995
(73) Patentinhaber: Kliegis, Ulrich Georg, D-24106 Kiel (DE)
(72) Erfinder: KLIEGIS, Ulrich, Georg, D-24106 Kiel (DE); LUNDT, Bernd, D-24143 Kiel (DE)
(74) Vertreter: Heldt, Gert, Dr. Dipl.-Ing.
(86) Internationale Anmeldenummer: DE9400157
(87) Internationale Veröffentlichungsnummer: WO9419758

(56) Entgegenhaltungen:
- EP-A- 0 326 768
- US-A- 5 215 095
- PROCEEDINGS OF COMPUTER BASED MEDICAL SYSTEMS, IEEE COMPUTER SOCIETY PRESS NEW YORK US 14. Juni 1992 , DURHAM NORTH CAROLINA US Seiten 309 - 314 XP000282957 P.F.HEMLER ET AL 'A THREE DIMENSIONAL GUIDANCE SYSTEM FOR FRAMELESS STEREOTATIC NEUROSURGERY'
- INDUSTRIAL ROBOT Bd. 20, Nr. 2 , 1993 , UK Seiten 28 - 29 P.A.FINLAY 'NEUROBOT: A FULLY ACTIVE ROBOT SYSTEM FOR ASSISTING IN NEUROSURGERY'
- PROCEEDING OF MEDICAL IMAGING, INTERNATIONAL COCIETY OF OPTICAL ENGINEERING US Bd. 767, Nr. 2 , 1. Februar 1987 , NEWPORT BEACH, CALIFORNIA US Seiten 509 - 514 B.A.KALL ET AL 'COMPREHENSIVE COMPUTER ASSISTED DATA COLLECTION TREATMENT PLANNING AND INTERACTIVE SURGERY'

## Beschreibung

Die Erfindung betrifft ein Verfahren zur Planung und Kontrolle eines chirurgischen Eingriffs in ein zuvor festgelegtes Operationsfeld, von dem nicht invasive Schnittbilder erzeugt werden, die zu einem dreidimensionalen Bild des Operationsfeldes zusammengefügt werden.

Darüberhinaus betrifft die Erfindung eine Vorrichtung zur Planung und Kontrolle eines chirurgischen Eingriffs mit einer Datenverarbeitungsanlage zur Erzeugung und Speicherung mindestens einer Abbildung eines Operationsfeldes aus zuvor erfaßten Strukturdaten.

Zur Vorbereitung komplizierter chirurgischer Eingriffe ist es üblich, die Vorgehensweise während des Eingriffs vorauszuplanen und den Eingriff zu simulieren, um dabei theoretisch die Schnittflächen festzulegen, an denen während des Eingriffs das Gewebe getrennt werden soll, wobei als Vorgabe möglichst wenig gesundes Gewebe entfernt werden und verletzliche Strukturen wie größere Gefäße oder Nerven weitgehend unberührt bleiben sollen. Dazu werden in der Regel zunächst ein Operationsfeld bestimmt und unter Zugrundelegen von Computertomographien, Röntgenbildern und anderen zuvor hergestellten Aufnahmen des Operationsfeldes optimale Schnittflächen bestimmt und potentielle Gefahrenbereiche in der Nähe der Schnittflächen diskutiert und auf den Aufnahmen vermerkt.

Da die der Planung der Schnittfläche zugrundeliegenden Aufnahmen jedoch zweidimensional sind, fordert eine Übertragung auf räumliche Verhältnisse ein großes Maß an Erfahrung und Vorstellungskraft. Zudem ist es schwierig, sich die oft komplexe Form der Schnittflächen einzuprägen, so daß während des Eingriffs vielfach unbeabsichtigt wieder von den optimalen Schnittflächen abgewichen wird.

Aus diesem Grund besteht insbesondere bei Osteotomien ein Bedürfnis, während des Eingriffs jederzeit auf die zuvor geplanten Schnittflächen zurückgreifen zu können, um einen Vergleich mit der tatsächlichen Schnittführung zu besitzen und um unbeabsichtigte Abweichungen von den geplanten Schnittflächen möglichst zu vermeiden.

Darüberhinaus ist aus der wissenschaftlichen Veröffentlichung "Proceeding of medical imaging, International society of optical engeneering, US, Bd. 767, Nr. 2, 1. Februar 1987, Newport Beach, Kalifornien US, Seiten 509 bis 514 von B. A. Kall et al.: Comprehensive computer assisted data collection treatment planning and interactive surgery" bekannt, Bezugspunkte auf einer Abbildung des Operationsfeldes festzulegen. Diese Bezugspunkte werden mit Hilfe einer Vorrichtung geschaffen, die - je nach der Verwendung des Meßverfahrens - auf den Umfang 9 bzw. 12 Stäbe verteilt hat. Die Stäbe einer Fläche besitzen die Konfiguration eines Buchstabens N, d. h. zwei der Stäbe einer Fläche verlaufen zueinander parallel, während der dritte innerhalb des von den beiden Parallelstäben gebildeten Raumes schräg verläuft. Durch die Zuordnung der drei Stäbe einer Seite kann festgelegt werden, in welcher Höhe beispielsweise eines Kopfes der von der Vorrichtung umgeben ist, sich ein auffälliges Merkmal, beispielsweise ein Krankheitsherd befindet.

Dieses Verfahren gibt dem die Operation durchführenden Chirurgen ein Maß dafür anhand, in welcher Tiefe er bezüglich einer vorgegebenen Bezugslinie innerhalb des Gewebes operieren muß. Dabei muß er eine Vielzahl von einzelnen Merkmalen bei der Durchführung des operativen Schnittes im Auge behalten, damit er sich nicht nur hinsichtlich der Tiefe, sondern auch der Breitenerstreckung des Operationsfeldes richtig orientieren kann. Darüberhinaus hat er auch noch in jeder einzelnen Ebene, die er bei der Durchführung des operativen Schnittes durchtrennt, auf besonders wichtige Teilaspekte Rücksicht zu nehmen, beispielsweise die Ebene kreuzende Gefäße oder Nerven.

Der Arzt muß daher nicht nur drei verschiedene Monitore beobachten, die ihm die jeweils aufgenommenen Meßergebnisse vermitteln, sondern darüberhinaus auch noch bei der Durchführung des operativen Schnittes seine Fachkenntnisse einsetzen, die ihm im Einzelfall überhaupt erst die Durchführung der Operation ermöglichen, da er aufgrund seines Fachwissens die ihm von den Monitoren vermittelten Daten über einzelne Punkte jeder Schnittfläche miteinander verbinden muß.

Aufgabe der vorliegenden Erfindung ist es daher, ein Verfahren und eine Vorrichtung zu entwickeln, mit welchen sich eine große Übereinstimmung zwischen einer geplanten und einer beim Eingriff vorgenommenen Schnittführung erzielen lassen.

Diese Aufgabe wird durch ein Verfahren nach Anspruch 1 und eine vorrichtung nach Anspruch 31 gelöst.

Diese Aufgabe wird beim Verfahren dadurch gelöst, daß in das dreidimensionale Bild des Operationsfeldes der Verlauf einer Schnittfläche eingefügt und in Form eines dreidimensionalen Bildes dargestellt wird, das einer Ansicht des Operationsteldes überlagert wird, und daß entsprechend der dargestellten Schnittfläche im Operationsfeld Gewebe voneinander getrennt wird.

Der Vorteil dieses Verfahrens besteht darin, daß eine Schnittfläche festgelegt wird, der der Chirurg mit seinem Operationsmesser folgen kann. Die Festlegung dieser Schnittfläche erfolgt unter Berücksichtigung der individuellen Voraussetzungen, die bei einem bestimmten Patienten zu berücksichtigen sind, d. h. unter anderem unter Beachtung des Verlaufs von Gefäßen und Nerven.

Das Bild des Operationsfeldes und der in diesem vorgesehenen Schnittfläche wird der Ansicht des Operationsfeldes überlagert. Auf diese Weise kann eine dreidimensionale Operationsplanung durchgeführt werden, die dreidimensionale Darstellung sichtbar gemacht und das so erzeugte Bild einem Realbild des Situs überlagert werden.

Dadurch erhält der Chirurg die Möglichkeit, den Verlauf des jeweils von ihm durchgeführten Schnittes bezüglich des von ihm vorgefundenen Operationsfeldes zu kontrollieren. Der von ihm durchgeführte Schnitt verändert mithin das von ihm vorgefundene Operationsfeld nicht so weitgehend, daß er die Wirkung des von ihm durchgeführten Schnittes auf dieses Operationsfeld nicht überprüfen kann. Vielmehr kann er durch einen Vergleich des von ihm vorgefundenen Operationsfeldes mit dem nach Durchführung des Schnittes erzeugten Operationsfeld feststellen, ob seine Schnittführung zu der von ihm gewünschten Veränderung des Operationsfeldes geführt hat und ob gegebenenfalls durch die Veränderung des Operationsfeldes Nachteile entweder entstanden oder zu befürchten sind, die für das Ergebnis der gesamten Operation vom Nachteil sind.

Gemäß einer bevorzugten Ausgestaltung der Erfindung wird der Planung der Schnittfläche eine dreidimensionale Soll-Abbildung zugrundegelegt, die rechnergestützt aus Strukturdaten erstellt wird, die man ebenfalls rechnergestützt durch die Auswertung von Computertomographie-, Kernspinresonanz-, Ultraschall-, Röntgen- oder holographischen Untersuchungen der damit erstellten Schichtaufnahmen des Operationsfeldes erhält. Diese dreidimensionale Soll-Abbildung kann beispielsweise auf einem Monitor dargestellt werden und ermöglicht es, die Anordnung der Schnittfläche über die gesamte Tiefe des Operationsfeldes zu planen.

Eine weitere bevorzugte Ausgestaltung der Erfindung sieht vor, daß beispielsweise durch Eingabe von Koordinaten von Punkten der Schnittfläche eine der Soll-Abbildung überlagerte dreidimensionale Abbildung der Schnittfläche auf dem Monitor erzeugt wird, wobei eine Optimierung dieser Schnittfläche durch Veränderung einzelner Parameter erfolgen kann und wobei mehrere Möglichkeiten der Schnittflächenanordnung als Datensatz gespeichert und bei Bedarf erneut abgerufen werden können. Eine vorteilhafte Ausgestaltung der Erfindung sieht vor, daß die dreidimensionale Abbildung der Schnittfläche als 3D-Datensatz gespeichert wird, so daß sie ebenfalls bei Bedarf abgerufen werden kann.

Während des Eingriffs kann dann die Soll-Abbildung zusammen mit den geplanten Schnittflächen oder nur die Schnittflächen selbst sowie außerdem Hinweislinien auf verletzliche Strukturen wie größere Gefäße, Nerven usw. im Sichtfeld des Chirurgen, beispielsweise auf einen Monitor oder einer Projektionsleinwand dargestellt oder auf das Operationsfeld selbst projiziert werden, um so dem Chirurgen als Leitlinie und Kontrolle beim Vornehmen der Gewebetrennschnitte zu dienen. Werden die Soll-Abbildung und die Schnittfläche auf einem Monitor oder auf einer Projektionsleinwand dargestellt, was gegenüber einer Projektion auf dem Operationsfeld selbst eine deutlichere Abbildung gewährleistet, so ist eine vollständige Kontrolle der tatsächlichen Schnittführung allerdings nur dann möglich, wenn dieser Soll-Abbildung, wie erfindungsgemäß vorgeschlagen wurde, eine Ist-Abbildung des Operationsfeldes unterlagert wird, so daß der Chirurg z.B. beim Durchtrennen von Gewebe in Echtzeit kontrollieren kann, ob er sein Instrument entlang der geplanten Schnittfläche führt.

Um eine koordinaten- und maßstabsgetreue Überlagerung der Schnittfläche, der Soll-Abbildung und der Ist-Abbildung zu gewährleisten, wird gemäß einer weiteren bevorzugten Ausgestaltung der Erfindung vorgeschlagen, die Schnittfläche und/oder die Soll-Abbildung mit der Ist-Abbildung zur Deckung zu bringen, indem das Operationsfeld beispielsweise mit einer Video-Kamera aufgenommen und das Videobildsignal einer Bildverarbeitungseinheit zugeführt wird, von wo es in Form eines Datensatzes einer Datenverarbeitungsanlage übermittelt wird, in der er in Echtzeit mit dem Datensatz der Soll-Abbildung verglichen wird, um Korrelationsfaktoren zu berechnen, die einen Ausgleich von Koordinatenverschiebungen und/oder Maßstabsdifferenzen ermöglichen. Anschließend wird dann die mit der Ist-Abbildung zur Deckung gebrachte Soll-Abbildung die Ist-Abbildung überlagernd auf dem Monitor dargestellt. Dabei kann auch manuell eine interaktive Anpassung der Perspektive vorgenommen werden.

Die Korrelation von Soll-Abbildung und Ist-Abbildung kann dadurch vereinfacht werden, daß gemäß einer vorteilhaften Ausgestaltung der Erfindung vor oder während des Eingriffs die Raumlage des Operationsfeldes in einem festgelegten Koordinatensystem vermessen und die Meßdaten in die Datenverarbeitungsanlage eingegeben werden.

Da die Ist-Abbildung des Operationsfeldes auch bei einer Darstellung auf einem Monitor oder einer Projektionsleinwand vorteilhafterweise ebenso wie die Soll-Abbildung bzw. die Abbildung der Schnittfläche dreidimensional ist, wird gemäß einer weiteren bevorzugten Ausgestaltung der Erfindung vorgeschlagen, für die Ist-Abbildung stereoskopische Bilder des Operationsfeldes zu erzeugen, die es dem mit einer Spezialbrille ausgestatteten Chirurgen erlauben, auch die Ist-Abbildung räumlich zu sehen. Eine derartige stereoskopische Darstellung kann beispielsweise dadurch erzeugt werden, daß das Operationsfeld von zwei nebeneinander angeordneten Videokameras aufgenommen wird, wobei auf dem Monitor oder der Projektionsleinwand abwechselnd in schneller Folge das Bild der einen bzw. der anderen Kamera abgebildet wird und wobei es eine synchron angesteuerte LCD-Brille, bei der in gleicher Folge abwechselnd das Sichtfeld jeweils eines Auges blockiert wird, dem Chirurgen ermöglicht, die Ist-Abbildung des Operationsfeldes als dreidimensionales Stereobild zu sehen.

Bei einer Projektion der Soll-Abbildung und der Schnittfläche unmittelbar auf das Operationsfeld können diese in ähnlicher Weise, beispielsweise durch Überlagerung zweier von entsprechend abgelenkten Laserstrahlen oder mit Hilfe von Videoprojektionsröhren erzeugten Kurvenzügen auf dem Operationsfeld als stereoskopische Projektion dargestellt und so bei Betrachtung mit einer Spezialbrille die Richtung der Schnittfläche in die Tiefe des Gewebes visuell sichtbar gemacht werden. Der Chirurg kann dann sein Skalpell ober ein anderes Schnittinstrument unmittelbar an der dreidimensional dargestellten Schnittfläche entlangführen. Im zuletzt genannten Fall ist es beispielsweise auch möglich, für die Soll-Abbildung eine Anaglyphendarstellung zu wählen, wobei dem Qperationsfeld ein Rot-Grün-Bild der Soll-Abbildung bzw. der Schnittfläche überlagert wird, welches beim Betrachten durch eine entsprechende Rot-Grün-Brille als stereoskopische Abbildung erscheint.

Vorteilhafterweise wird sowohl für die Soll-Abbildung als auch für die Abbildung der Schnittfläche eine Darstellung aus einer Richtung gewählt, die im wesentlichen der Blickrichtung des Chirurgen während des Eingriffs entspricht. Eine weitere Möglichkeit besteht darin, die Soll-Abbildung bzw. die Schnittfläche stets als Draufsicht von oben abzubilden, da die Abbildung dann von der Position des Chirurgen bezüglich des Operationsfeldes unabhängig ist.

Eine weitere vorteilhafte Ausgestaltung der Erfindung sieht vor, daß die Lage des vom Chirurgen während des Eingriffs geführten Instruments fortlaufend ermittelt und in der mit der Ist-Abbildung zur Deckung gebrachten Soll-Abbildung mit der Lage der Schnittfläche verglichen wird. Abweichungen des Instruments von der geplanten Schnittfläche können dem Chirurgen beispielsweise durch ein akustisches oder optisches Signal mitgeteilt werden, worauf hin er die Schnittführung, falls gewünscht, korrigieren kann. Alternativ zur Anzeige der Abweichung kann auch die Bewegung des Instruments entlang der geplanten Schnittfläche durch eine von der Datenverarbeitungsanlage gesteuerte Instrumentenführung unterstützt werden, die bei Abweichungen der Ist-Position des Instruments von der Soll-Position der vom Chirurgen auf das Instrument ausgeübten Kraft eine entsprechend berechnete Kraft entgegensetzt, die eine Rückkehr des Instruments auf die geplante Schnittfläche bewirkt oder das Instrument innerhalb eines vorgegebenen Toleranzbereichs zur Schnittfläche hält. Die Wirkung der Instrumentenführung ist zweckmäßig in ihrem Einfluß steuerbar, so daß der Chirurg auf einen gegenüber dem simulierten Eingriff unterschiedlich verlaufenden Eingriff rasch reagieren kann.

Im Hinblick auf die Vorrichtung wird die der Erfindung zugrundeliegende Aufgabe dadurch gelöst, daß in der Datenverarbeitungsanlage eine Abbildung einer sich durch die Abbildung des Operationsfeldes erstreckenden Schnittfläche erzeugbar und speicherbar ist, entlang der ein Gewebe des Operationsfeldes trennbar ist, und eine Bildwiedergabevorrichtung zur Projizierung einer auf diese Weise erzeugten Soll-Abbildung auf eine Ansicht des Operationsfeldes vorgesehen ist.

Diese Vorrichtung ist dazu geeignet, die Soll-Abbildung mit einer Ansicht des Operationsfeldes in räumliche Übereinstimmung zu bringen und auf diese Weise eine optimale Schnittführung durchzuführen. Dadurch wird der Chirurg in die Lage versetzt, mit großer Sicherheit auch sehr schwierige Schnitte durchzuführen.

Um die Soll-Abbildung mit dem Operationsfeld selbst exakt zur Deckung zu bringen, sieht eine bevorzugte Ausgestaltung der Erfindung eine Vorrichtung zum Vermessen des Operationsfeldes vor, deren Meßdaten in die Datenverarbeitungsanlage eingegeben werden können, um eine Korrelation von Maßstab und Koordinaten der Soll-Abbildung und des Operationsfelds zu erzeugen.

Die der Erfindung zugrundeliegende Aufgabe kann auch durch eine Vorrichtung gelöst werden, die mindestens umfaßt: ein Bildaufnahmegerät zum Herstellen einer vorzugsweise kontinuierlichen Ist-Abbildung des Operationsfelds, eine Datenverarbeitungsanlage (DVA), von welcher mindestens eine Soll-Abbildung des Operationsfeldes aus zuvor erfaßten Strukturdaten erzeugt, gemeinsam mit mindestens einer auf der Grundlage der Soll-Abbildung geplanten Schnittfläche gespeichert und mit der Ist-Abbildung zur Deckung gebracht werden kann, sowie mindestens eine Bildwiedergabevorrichtung für die Darstellung der der Ist-Abbildung überlagerten Schnittfläche und/oder Soll-Abbildung. Eine vorteilhafte Ausgestaltung der Erfindung sieht weiter eine Vorrichtung zum Einlesen von Koordinaten, beispielsweise einen Scanner, mindestens einer die Soll-Abbildung überlagernd dargestellten Schnittfläche vor.

An das Bildaufnahmegerät ist zweckmäßig eine Bildverarbeitungseinheit angeschlossen, von welcher das aufgezeichnete Bild des Operationsfelds in elektronische Daten umgewandelt und an die DVA übermittelt wird, wo es mit dem Datensatz der Soll-Abbildung verglichen wird, um die Soll-Abbildung und die Schnittfläche mit der Ist-Abbildung im Hinblick auf den Koordinatennullpunkt, die Richtung der Koordinatenachsen und den Maßstab zur Deckung zu bringen.

Eine weitere vorteilhafte Ausgestaltung der Erfindung sieht mindestens zwei Bildaufnahmegeräte zum Aufzeichnen von stereoskopischen Ist-Abbildungen des Operationsfelds vor, die einer dreidimensionalen Abbildung der Schnittfläche und/oder dreidimensionalen Soll-Abbildung unterlagert werden können.

Gemäß einer weiteren vorteilhaften Ausgestaltung der Erfindung ist die Bildwiedergabevorrichtung als Monitor ausgebildet und während des Eingriffs im Sichtbild des Chirurgen angeordnet.

Im folgenden wird die Erfindung anhand eines Ausführungsbeispieles näher erläutert.

### Ausführungsbeispiel

Zur Planung und Vorbereitung eines chirurgischen Eingriffs zur Beseitigung eines Tumors in einem menschlichen Schädel wird als erstes mit Hilfe eines Computertomographen und/oder eines Kernspintomographen eine Serie von Schnittbildaufnahmen des Schädels erstellt, wobei die Aufnahmen jeweils eine Schicht abbilden und die Schichten in geringem Abstand parallel übereinander liegen. Dabei werden die durch die Tomographie ermittelten Meßdaten einer Datenverarbeitung zugeleitet, in welcher daraus mittels entsprechender Software nach Festlegen eines Operationsfelds eine dreidimensionale Soll-Abbildung desselben erstellt wird, d.h. des den Tumor aufweisenden Schädelbereichs. Zusätzlich können anschließend weitere Strukturdaten eingegeben werden, welche beispielsweise durch Auswertung der Ergebnisse von Ultraschall-, Röntgen- oder holographischen Untersuchungen des Schädels erhalten wurden.

Die dreidimensionale Abbildung des Operationsfeldes wird anschließend als Graphik auf einem Monitor abgebildet, und dient als Grundlage für die Planung bzw. Simulation des Eingriffs.

Bei der Planung des Eingriffs werden unter Berücksichtigung der Ergebnisse der zuvor genannten Untersuchungen die krankhaft veränderten Teile des Gehirns ermittelt und Schnittflächen festgelegt, an denen während des Eingriffs das Gewebe getrennt werden soll. Dabei werden die Schnittflächen so gelegt, daß zum einen möglichst wenig gesundes Gewebe entfernt wird, und daß zum anderen möglichst wenige größere Gefäße verletzt werden. Um das letztere zu erreichen, können in der Nähe der Schnittfläche liegende Gefäße in der dreidimensionalen Soll-Abbildung farblich hervorgehoben oder durch Hinweislinien gekennzeichnet werden.

Um die Schnittflächen selbst der Soll-Abbildung überlagernd darzustellen, können beispielsweise Koordinaten der Schnittfläche in einen der Soll-Abbildung auf dem Monitor überlagerten Koordinatensystem bestimmt und durch Eingabe der Koordinaten in die Datenverarbeitungsanlage (DVA) die Schnittflächen farblich abgehoben oder als transparente oder durchscheinende Flächen in die Soll-Abbildung aufgenommen werden, wobei jedoch zweckmäßig auch ein Datensatz erstellt wird, der ausschließlich die Schnittflächen im Maßstab und im Koordinatensystem der Soll-Abbildung enthält.

Eine weitere Möglichkeit zum Eingeben der Schnittflächen besteht darin, die dreidimensionale Abbildung des Operationsfeldes in zweidimensionale Schichtbilder zu zerlegen. Dabei können nicht nur plane sondern auch geschwungene Schnittflächen eingegeben werden. In die zweidimensionalen Schnittbilder wird dann jeweils mit einem grafischen Eingabemedium - wie z.B. einem Grafiktablett - ein linearer Kurvenzug eingegeben, der die Schnittfläche in dem entsprechenden Schichtbild wiedergibt. Aus der Summe der eingelesenen Schnittlinien kann dann wieder eine dreidimensionale, die Soll-Abbildung überlagernde Schnittflächenabbildung erstellt werden.

Um eine optimale Schnittführung zu erhalten, kann die dreidimensionale Soll-Abbildung beispielsweise im Raum gedreht und Ausschnitte vergrößert oder verkleinert werden.

Nach Beendigung der Planung des Eingriffs werden der 3D-Datensatz der soll-Abbildung und der Abbildung der Schnittflächen gespeichert, wobei die Schnittflächen zweckmäßigerweise in einzelne Flächenelemente zerlegt gespeichert werden, welche sowohl getrennt als auch gemeinsam abgerufen werden können.

Vor Beginn des eigentlichen Eingriffs wird dann der Kopf des Patienten auf einer Auflage unter einem Operationsmikroskop in allen Richtungen unverschiebbar fixiert und gegenüber einem feststehenden Koordinatensystem der Auflage vermessen, wobei die Raumkoordinaten bestimmter charakteristischer Punkte des Schädels bestimmt werden.

Diese Koordinatenpunkte werden der Datenverarbeitungsanlage eingegeben und Korrelationsfaktoren berechnet, um das Koordinatensystem der Soll-Abbildung und der Schnittflächen mit dem feststehenden Koordinatensystem der Auflage zur Deckung zu bringen.

Am Operationsmikroskop angebrachte Sensoren ermitteln die Blickrichtung durch das Mikroskop auf das Operationsfeld sowie die jeweils eingestellte Vergrößerung und übertragen diese Daten ebenfalls zur Datenverarbeitungsanlage.

Aus diesen Daten wird anschließend eine korrigierte Soll-Abbildung und eine korrigierte Abbildung der Schnittflächen erstellt, welche im Hinblick auf ihren Koordinatennullpunkt, die Richtungen der Koordinatenachsen, den Maßstab und die Blickrichtung genau der im Okular des Operationsmikroskops sichtbaren Ist-Abbildung entspricht.

Die korrigierte Abbildung der Schnittflächen bzw. die korrigierte Soll-Abbildung kann anschließend so in den Strahlengang des Operationsmikroskopes eingespiegelt werden, daß sie die im Okular sichtbare Ist-Abbildung überlagert. Dabei können die korrigierte Soll-Abbildung und die korrigierte Abbildung der Schnittflächen auf einer Bildwiedergabevorrichtung dargestellt und das wiedergebene Bild dann derart in den Strahlengang des Operationsmikroskops eingespiegelt werden, daß es die Ist-Abbildung überlagernd durch das Okular auf das Auge des Chirurgen fällt.

Bei einem als Stereo-Mikroskop ausgebildeten. Qperationsmikroskop können entsprechend zwei korrigierte Soll-Abbildungen und korrigierte Abbildungen der Schnittfläche erzeugt werden, die der Blickrichtung durch jedes der Okulare entsprechen. Durch Einspiegelung der korrigierten Abbildungen in das jeweilige Okular kann der stereokopischen Ist-Abbildung des Operationfeldes eine stereoskopische Soll-Abbildung bzw. eine stereoskopische Darstellung der Schnittflächen überlagert werden, wobei die Richtung der Schnittflächen oder von Elementen der Schnittfläche in die Tiefe der Gewebestruktur visuell sichtbar ist. Der Chirurg kann dann dementsprechend sein Instrument im Operationsfeld entsprechend der dargestellten visuellen Informationen positionieren und bewegen.

Die Kontrolle der Schnittführung kann dadurch noch weiter verbessert werden, daß das durch das Okular des Operationsmikroskopes sichtbare Operationsfeld kontinuierlich mit einer Videokamera aufgezeichnet und die aufgezeichneten Bilder in Echtzeit einer Bildverarbeitungseinheit zugeführt werden, wobei die Daten vom Ausgang der Bildverarbeitungseinheit der Datenverarbeitungsanlage zugeführt und dort kontinuierlich mit der Abbildung der Schnittflächen verglichen werden. Bei Abweichungen des chirurgischen Instruments von der vorgeplanten Schnittfläche kann der Chirurg dann mittels eines optischen oder akustischen Warnsignals auf die Abweichungen hingewiesen werden. Weiter kann eine in Echtzeit von der Datenverarbeitungsanlage gesteuerte Instrumentenführung für das vom Chirurgen verwendete Instrument eingesetzt werden, die dem Chirurgen die Ausführung aller Bewegungen überläßt, ihn jedoch auf Bewegungspfade eingrenzt die der geplanten Schnittführung mit gewissen Toleranzen entsprechen.

Die Instrumentenführung kann dabei z.B. über einen Servomechanismus mit elektrischer, pneumatischer oder hydraulischer Betätigung mit einer entsprechend der Größe der Abweichungen variierenden, der vom Chirurgen ausgeübten Schnittkraftkomponente entgegengesetzten Kraftkomponente beaufschlagt werden.

Besondere Bedeutung hat dieses Verfahren bei, der Resektion erkrankter Knochenbezirke, in die anschließend ein vorgefertiges individuelles Implantat eingesetzt werden soll. Die durch ein Herstellungsverfahren des Implantats unter Verwendung von CAD oder durch Abtastung erhaltenen geometrische Daten der Grenzflächen des Implantats zum Knochen hin stellen in diesem Fall die Schnittflächen dar, die durch die Osteotomie dargestellt werden müssen.

## Patentansprüche

1. Verfahren zur Planung und Kontrolle eines chirurgischen Eingriffs in ein zuvor festgelegtes Operationsfeld, von dem nicht invasive Schnittbilder erzeugt werden, die zu einem dreidimensionalen Bild des Operationsfeldes zusammengefügt werden, dadurch gekennzeichnet, daß in das dreidimensionale Bild des Operationsfeldes der Verlauf einer Schnittfläche eingefügt und in Form eines dreidimensionales Bildes dargestellt wird, das einer Ansicht des Operationsfeldes überlagert wird, und daß entsprechend der dargestellten Schnittfläche im Operationsfeld Gewebe voneinander getrennt wird.

2. Verfahren nach Anspruch 1, dadurch gekennzeichnet, daß eine Darstellung der Schnittfläche während der Operation auf das Operationsfeld projiziert wird.

3. Verfahren nach Anspruch 1, dadurch gekennzeichnet, daß die Abbildung der Schnittfläche einem intraoperativ erzeugten Realbild der Körperregion überlagert wird.

4. Verfahren nach einem der Ansprüche 1 bis 3, dadurch gekennzeichnet, daß eine das geplante Operationsfeld wiedergebende Soll-Abbildung rechnergestützt aus Daten unterschiedlich erzeugter Aufnahmen erstellt wird.

5. Verfahren nach Anspruch 4, dadurch gekennzeichnet, daß die Aufnahmen das Operationsfeld in jeweils übereinanderliegenden Schichten abbilden.

6. Verfahren nach einem der Ansprüche 1 bis 5, dadurch gekennzeichnet, daß beim Erstellen der Soll-Abbildung Computertomographien, Kernspinresonanz-, Ultraschall-, und/oder Röntgenaufnahmen des Operationsfeldes ausgewertet werden.

7. Verfahren nach einem der Ansprüche 1 bis 6, dadurch gekennzeichnet, daß die Soll-Abbildung als Datensatz im Speicher einer DVA gespeichert wird.

8. Verfahren nach einem der Ansprüche 1 bis 7, dadurch gekennzeichnet, daß die Soll-Abbildung zur Planung des Eingriffs auf einer Bildwiedergabevorrichtung abgebildet wird.

9. Verfahren nach einem der Ansprüche 1 bis 8, dadurch gekennzeichnet, daß die Abbildung der Schnittfläche der Soll-Abbildung überlagert wird.

10. Verfahren nach einem der Ansprüche 1 bis 9, dadurch gekennzeichnet, daß sich die Schnittfläche visuell von der Soll-Abbildung abhebt.

11. Verfahren nach einem der Ansprüche 1 bis 10, dadurch gekennzeichnet, daß die Schnittfläche von transparenten oder semitransparenten Linien begrenzt wird oder als Fläche dargestellt wird.

12. Verfahren nach einem der Ansprüche 1 bis 11, dadurch gekennzeichnet, daß die Abbildung der Schnittfläche als Datensatz gespeichert wird.

13. Verfahren nach einem der Ansprüche 1 bis 12, dadurch gekennzeichnet, daß eine dreidimensionale Abbildung der Schnittfläche als 3D-Datensatz gespeichert wird.

14. Verfahren nach einem der Ansprüche 1 bis 13, dadurch gekennzeichnet, daß die Abbildung der geplanten Schnittfläche mit einer vor Beginn des Eingriffs aufgenommenen Ist-Abbildung des Operationsfeldes zur Deckung gebracht wird.

15. Verfahren nach einem der Ansprüche 1 bis 14, dadurch gekennzeichnet, daß ein Bild des Operationfelds aus dem Strahlengang eines Operationsmikroskops einer Bildverarbeitungseinheit zugeführt und von dort als Datensatz einer DVA übermittelt wird.

16. Verfahren nach einem der Ansprüche 1 bis 14, dadurch gekennzeichnet, daß der Bereich des Operationsfeldes von mindestens einer Videokamera aufgenommen wird, und daß die aufgenommenen Bilder einer Bildverarbeitungseinheit zugeführt und von dort als Datensatz einer DVA übermittelt werden.

17. Verfahren nach Anspruch 15 oder 16, dadurch gekennzeichnet, daß kontinuierlich Bilder des Operationsfeldes der Bildverarbeitungseinheit zugeführt werden.

18. Verfahren nach einem der Ansprüche 15 bis 17, dadurch gekennzeichnet, daß mit Hilfe zweier nebeneinander angeordneter Videokameras stereoskopische Bilder des Operationsfeldes erzeugt werden.

19. Verfahren nach einem der Ansprüche 15 bis 18, dadurch gekennzeichnet, daß der von der Bildverarbeitungseinheit übermittelte Datensatz in der DVA mit dem Datensatz der Soll-Abbildung verglichen wird.

20. Verfahren nach einem der Ansprüche 1 bis 19, dadurch gekennzeichnet, daß vor dem Eingriff die Raumlage des Operationsfeldes in einem festgelegten Koordinatensystem vermessen wird.

21. Verfahren nach einem der Ansprüche 1 bis 20, dadurch gekennzeichnet, daß Korrelationsfaktoren berechnet werden, um die Koordinatensysteme und/oder den Maßstab von Ist- und Soll-Abbildung zur Deckung zu bringen.

22. Verfahren nach Anspruch 21, dadurch gekennzeichnet, daß eine entsprechend korrigierte, mit der Ist-Abbildung zur Deckung gebrachte Abbildung der Schnittfläche rechnergestützt erzeugt wird.

23. Verfahren nach Anspruch 20 oder 21, dadurch gekennzeichnet, daß anschließend die korrigierte Abbildung der Schnittfläche in den Strahlengang des Operationsmikroskops eingespiegelt wird.

24. Verfahren nach Anspruch 20 oder 21, dadurch gekennzeichnet, daß anschließend die korrigierte Abbildung der Schnittfläche auf einer Bildwiedergabeeinrichtung die Ist-Abbildung überlagernd wiedergegeben wird.

25. Verfahren nach Anspruch 20 oder 21, dadurch gekennzeichnet, daß anschließend die korrigierte Abbildung der Schnittfläche auf den Bereich des Operationsfeldes projiziert wird.

26. Verfahren nach einem der Ansprüche 1 bis 25, dadurch gekennzeichnet, daß die projizierte Schnittfläche von einer den Eingriff vornehmenden Person mittels einer ein Stereobild der Schnittfläche erzeugenden Vorrichtung betrachtet wird.

27. Verfahren nach einem der Ansprüche 1 bis 26, dadurch gekennzeichnet, daß die Raumlage mindestens eines beim Eingriff verwendeten chirurgischen Instrumentes gegenüber dem festgelegten Koordinatensystem kontinuierlich oder intermittierend gemessen wird.

28. Verfahren nach Anspruch 27, dadurch gekennzeichnet, daß bei Abweichungen des Instruments von der Schnittfläche ein akustisches oder optisches Warnsignal betätigt wird.

29. Verfahren nach Anspruch 27 oder 28, dadurch gekennzeichnet, daß die Führung des Instruments entlang der Schnittfläche durch eine von der DVA gesteuerte Instrumentenführung unterstützt wird.

30. Verfahren nach einem der Ansprüche 1 bis 29, dadurch gekennzeichnet, daß neben der Schnittfläche mindestens eine Hinweislinie auf verletzliche Gewebeteile wie Gefäße, Nerven oder dergleichen und/oder mindestens eine Resektionslinie als Datensatz gespeichert und die Ist-Abbildung überlagernd wiedergegeben oder auf das Operationsfeld projiziert wird.

31. Vorrichtung zur Planung und Kontrolle eines chirurgischen Eingriffs, insbesondere zur Durchführung des Verfahrens nach einem der Ansprüche 1 bis 30 mit einer Datenverarbeitungsanlage zur Erzeugung und Speicherung mindestens einer Abbildung eines Operationsfeldes aus zuvor erfaßten Strukturdaten, dadurch gekennzeichnet, daß in der Datenverarbeitungsanlage eine Abbildung einer sich durch das dreidimensionale Bild des Operationsfeldes erstreckenden Schnittfläche erzeugbar und speicherbar ist, entlang der im Operationsfeld Gewebe voneinander trennbar ist, und eine Bildwiedergabevorrichtung zur Projizierung einer auf diese Weise erzeugten Soll-Abbildung auf eine Ansicht des Operationsfeldes vorgesehen ist.

32. Vorrichtung nach Anspruch 31, dadurch gekennzeichnet, daß die Eildwiedergabevorrichtung zur Projizierung der Soll-Abbildung während der Operation auf das Operationsfeld ausgerichtet ist.

33. Vorrichtung nach Anspruch 31, dadurch gekennzeichnet, daß die erzeugte Soll-Abbildung einem intraoperativ erzeugten Realbild des Operationsfeldes elektronisch überlagert wird.

34. Vorrichtung nach einem der Ansprüche 31 bis 33, gekennzeichnet durch eine Einrichtung zum Vermessen des Operationsfelds.

35. Vorrichtung nach einem der Ansprüche 31 bis 34, dadurch gekennzeichnet, daß die Bildwiedergabevorrichtung zwei einander in schneller Folge überlagerte Halbbilder erzeugt.

36. Vorrichtung nach Anspruch 35, gekennzeichnet durch eine mit der Bildwiedergabevorrichtung synchron gesteuerte stereoskopische Videodarstellungseinheit für die den Eingriff durchführende Person.

37. Vorrichtung nach einem der Ansprüche 31 bis 36, gekennzeichnet durch einen Laser zum Projizieren der Soll-Abbildung und/oder der Abbildung der Schnittfläche.

38. Vorrichtung nach einem der Ansprüche 31 bis 37, gekennzeichnet durch zwei Videoprojektionsröhren zum Projizieren der Soll-Abbildung und/oder der Abbildung der Schnittfläche.

39. Vorrichtung nach einem der Ansprüche 31 bis 38, gekennzeichnet durch eine von der DVA gesteuerte Ablenkvorrichtung zum Ablenken der projizierten Abbildung.

40. Vorrichtung nach Anspruch 31, gekennzeichnet durch mindestens ein Bildaufnahmegerät zum Herstellen einer kontinuierlichen Ist-Abbildung des Operationsfeldes, die mit mindestens einer von der Datenverarbeitungsanlage (DVA) aus zuvor erfaßten Strukturdaten erzeugten und gemeinsam mit mindestens einer geplanten Schnittfläche gespeicherten Soll-Abbildung des Operationsfeldes zur Deckung gebracht werden kann, sowie durch mindestens eine Bildwiedergabevorrichtung für die Darstellung der der Ist-Abbildung überlagerten Schnittfläche und/oder Soll-Abbildung.

41. Vorrichtung nach einem der Ansprüche 31 bis 40, gekennzeichnet durch eine Vorrichtung zum Einlesen von Koordinaten mindestens einer die Soll-Abbildung überlagernd dargestellten Schnittfläche.

42. Vorrichtung nach Anspruch 40 oder 41, gekennzeichnet durch mindestens zwei Bildaufnahmegeräte zum Aufzeichnen einer stereoskopischen Ist-Abbildung des Operationsfelds.

43. Vorrichtung nach einem der Ansprüche 40 bis 42, gekennzeichnet durch eine an das Bildaufnahmegerät angeschlossene Bildverarbeitungseinheit.

44. Vorrichtung nach einem der Ansprüche 40 bis 43, dadurch gekennzeichnet, daß die Bildwiedergabevorrichtung im Sichtfeld einer den Eingriff durchführenden Person angeordnet ist.

45. Vorrichtung nach einem der Ansprüche 40 bis 44, dadurch gekennzeichnet, daß die Bildwiedergabevorrichtung ein Monitor ist.

46. Vorrichtung nach einem der Ansprüche 31 bis 45, gekennzeichnet durch ein Operationsmikroskop mit Mitteln zum Einspiegeln der Soll-Abbildung und/oder der Schnittfläche in den Strahlengang des Mikroskops.

47. Vorrichtung nach einem der Ansprüche 31 bis 46, gekennzeichnet durch mindestens ein mit einer Meßvorrichtung zum Messen seiner Koordinatenlage gekoppeltes chirurgisches Instrument.

48. Vorrichtung nach einem der Ansprüche 31 bis 47, gekennzeichnet durch mindestens ein mit einer von der DVA gesteuerten Instrumentenführung gekoppeltes chirurgisches Instrument.

## Claims

1. Method for planning and monitoring a surgical operation in a previously determined operating area, of which non-invasive sectional views are generated that are combined to form a three-dimensional image of said operating area, characterised in that into the three-dimensional image of the operating area, the path of an incision region is inserted, and is represented in the form of a three-dimensional image which is superimposed onto a view of the operating area, and that tissue is separated in accordance with the incision region shown in the operating area.

2. Method according to claim 1, characterised in that a representation of the incision region is projected onto the operating are a during the operation.

3. Method according to claim 1, characterised in that the image of the incision region is superimposed with an intra-operatively generated actual image of the region of the body.

4. Method according to one of claims 1 to 3, characterised in that a reference image reproducing the planned operating area is prepared with computer assistance using data from differently generated recordings.

5. Method according to claim 4, characterised in that the recordings form an image of the operating area in respectively superimposed layers.

6. Method according to one of claims 1 to 5, characterised in that when the reference image is prepared, computer tomography, nuclear magnetic resonance, ultrasound and/or X-ray images of the operating area are analysed.

7. Method according to one of claims 1 to 6, characterised in that the reference image is stored as a data record in the memory of a computer.

8. Method according to one of claims 1 to 7, characterised in that the reference image for planning the operation is formed on an image display device.

9. Method according to one of claims 1 to 8, characterised in that the image of the incision region is superimposed with the reference image.

10. Method according to one of claims 1 to 9, characterised in that the incision region stands out visually from the reference image.

11. Method according to one of claims 1 to 10, characterised in that the incision region is delimited by transparent or semi-transparent lines or is shown as a surface.

12. Method according to one of claims 1 to 11, characterised in that the image of the incision region is stored as a data record.

13. Method according to one of claims 1 to 12, characterised in that a three-dimensional image of the incision region is stored as a 3-D data record.

14. Method according to one of claims 1 to 13, characterised in that die image of die planned incision region is made to coincide with an actual image of the operating area recorded before the beginning of the operation.

15. Method according to one of claims 1 to 14, characterised in that an image of die operating area from the optical path of a surgical microscope is supplied to an image processor and is transmitted in die form of a data record from there to a Computer.

16. Method according to one of claims 1 to 14, characterised in that the region of the operating area is recorded by at least one video camera and that the recorded images are supplied to an image processor and from there are transmitted as a data record to a Computer.

17. Method according to claim 15 or 16, characterised in that images of the operating area are continuously supplied to the image processor.

18. Method according to one of claims 15 to 17, characterised in that stereoscopic images of the operating area are generated with the aid of two adjacently arranged video cameras.

19. Method according to one of claims 15 to 18, characterised in that the data record transmitted from the image processor to the computer is compared to the data record of the reference image.

20. Method according to one of claims 1 to 19, characterised in that prior to the operation, the spatial position of the operating area is measured with in an established coordinate system.

21. Method according to one of claims 1 to 20, characterised in that correlation factors are calculated in order to make the coordinate system and/or the scale of the actual and reference images coincide.

22. Method according to claim 21, characterised in that a correspondingly corrected image of the incision region made to coincide with the actual image is created with computer assistance.

23. Method according to claim 20 or 21, characterised in that subsequently, the corrected image of the incision region is reflected into the optical path of the surgical microscope.

24. Method according to claim 20 or 21, characterised in that subsequently, the corrected image of the incision region is reproduced on an image display device, superimposing the actual image.

25. Method according to claim 20 or 21, characterised in that subsequently the corrected image of the incision region is projected onto the region of the operating area.

26. Method according to one of claims 1 to 25, characterised in that the projected incision region is viewed by a person undertaking the operation by means of a device generating a stereo image of the incision region.

27. Method according to one of claims 1 to 26, characterised in that the spatial position of at least one surgical instrument used during the operation is continuously or intermittently measured with respect to the established coordinate system.

28. Method according to claim 27, characterised in that an acoustic or optical alarm signal is actuated when the instrument deviates from the incision region.

29. Method according to claim 27 or 28, characterised in that guiding of the instruments along the incision region is assisted by computer-controlled instrument guidance.

30. Method according to one of claims 1 to 29, characterised in that in addition to the incision region, at least one line indicating injured tissues such as vessels, nerves or the like and/or at least one resection line is stored as a data record, and is reproduced superimposing the actual image, or projected onto the operating area.

31. Device for planning and monitoring a surgical operation, in particular for carrying out the method according to one of claims 1 to 30, with a computer for generating and storing at least one image of an operating area from previously recorded configuration data, characterised in that an image of an incision region extending through the three-dimensional image of the operating area can be generated by and stored in the computer, along which incision surface tissue can be separated in the operating area, and an image display device is provided for projecting a reference image generated in this way onto a view of the operating area

32. Device according to claim 31, characterised in that the image display device is aligned to project the reference image onto the operating area during the operation.

33. Device according to claim 31, characterised in that the reference image generated is electronically superimposed with an intra-operatively generated actual image of the operating area.

34. Device according 10 one of claims 31 to 33, characterised by a means for measuring the operating area.

35. Device according to one of claim 31 to 34, characterised in that the image display device generates two half-frames superimposed in rapid succession.

36. Device according to claim 35, characterised by a stereoscopic video display unit controlled synchronously with the image display device for the person carrying out the operation.

37. Device according to one of claims 31 to 36, characterised by a laser for projecting the reference image and/or the image of the incision region.

38. Device according to one of claims 31 to 37, characterised by two video projection tubes for projecting the reference image and/or the image of the incision region.

39. Device according to one of claims 31 to 38, characterised by a computer controlled defleetor for deflecting the projected image.

40. Device according to claim 31, characterised by at least one image recording device for producing it continuous actual image of the operating area, which image can be made to coincide with at least one reference image of the operating area generated by the computer from configuration data previously captured and stored together with at least one scheduled incision region, as well as by at least one image display device for showing the incision region superimposed with the actual image, and/or reference image.

41. Device according to one of claims 31 to 40, characterised by a device for inputting coordinates of at least one incision region shown superimposing the reference image.

42. Device according to claim 40 or 41; characterised by at least two image recording devices tor displaying a stereoscopic actual image of the operating area.

43. Device according to one of claims 40 to 42, characterised by an image processor connected to the image recording device.

44. Device according to one of claims 40 to 43, characterised in that the image display device is arranged in the field of view of a person carrying out the operation.

45. Device according to one of claims 40 to 44, characterised in that image display device is a monitor.

46. Device according to one of claims 31 to 45, characterised by a surgical microscope with means for reflecting the reference image ad/or the incision area into the optical path of the microscope.

47. Device according to one of claims 31 to 46, characterised by at least one surgical instrument coupled to a measuring device for measuring the coordinate position of said instrument.

48. Device according to one of claims 31 to 47, characterised by at least one surgical instrument coupled to an instrument guide controlled by the computer.

## Revendications

1. Procédé pour la planification et le contrôle d'une intervention chirurgicale dans un champ opératoire préalablement défini, pour lequel des images d'incision sont produites de manière non invasive et assemblées pour former une image tridimensionnelle du champ opératoire, caractérisé par le fait qu'on insère dans l'image tridimensionnelle du champ opératoire le tracé d'une surface d'incision et on la représente sous la forme d'une image tridimensionnelle qui est superposée à une vue du champ opératoire et par le fait qu'on sépare les tissus dans le champ opératoire selon la surface d'incision représentée.

2. Procédé selon la revendication 1, caractérisé par le fait qu'on projette une représentation de la surface d'incision sur le champ opératoire, pendant l'opération.

3. Procédé selon la revendication 1, caractérisé par le fait que l'on superpose la représentation de la surface d'incision à une image réelle de la zone du corps produite de manière intra-opératoire.

4. Procédé selon une des revendications 1 à 3, caractérisé par le fait que l'on produit de manière assistée par ordinateur une représentation théorique reproduisant le champ opératoire planifié, à partir de données provenant de clichés produits de différentes manières.

5. Procédé selon la revendication 4, caractérisé par le fait que les clichés représentent le champ opératoire dans des couches disposées les unes au-dessus des autres.

6. Procédé selon une des revendications 1 à 5, caractérisé par le fait que pour la production de la représentation théorique on exploite des images du champ opératoire obtenues par tomographie par ordinateur, par résonance magnétique nucléaire, par échographie et/ou par radiagraphie.

7. Procédé selon une des revendications 1 à 6, caractérisé par le fait que la représentation théorique est mémorisée sous forme de jeu de données dans la mémoire d'une installation de traitement de données.

8. Procédé selon une des revendications 1 à 7, caractérisé par le fait que la représentation théorique, pour la planification de l'intervention, est reproduite sur un dispositif de reproduction vidéo.

9. Procédé selon une des revendications 1 à 8, caractérisé par le fait que l'on superpose la représentation de la surface d'incision à la représentation théorique.

10. Procédé selon une des revendications 1 à 9, caractérisé par le fait que la représentation de la surface d'incision se détache sur le plan visuel par rapport la représentation théorique.

11. Procède selon une des revendications 1 à 10, caractérisé par le fait que la représentation de la surface d'incision est délimitée par des lignes transparentes ou semi-transparentes et est représentée en tant que surface.

12. Procédé selon une des revendications 1 à 11, caractérisé par le fait que la représentation de la surface d'incision est mémorisée sous forme de jeu de données.

13. Procédé selon une des revendications 1 à 12, caractérisé par le fait qu'une représentation tridimensionnelle de la surface d'incision est mémorisée sous forme de jeu de données tridiemensionnelles.

14. Procédé selon une des revendications 1 à 13, caractérisé par le fait quel'on amène la représentation de la surface d'incision planifiée en recouvrement avec une représentation réelle du champ opératoire enregistrée avant le début de l'intervention.

15. Procédé selon une des revendications 1 à 14, caractérisé par le fait qu'on envoie à une unité de traitement d'image une image du champ opératoire prélevée sur la marche des rayons d'un microscope d'opération et, de là, on la transmet sous forme de jeu de données à une installation de traitement de données.

16. Procédé selon une des revendications 1 à 14, caractérisé par le fait qu'on enregistre la zone du champ opératoire avec au moins une caméra vidéo et qu'on transmet les images enregistrées à une unité de traitement d'images et, de là, on la transmet sous forme de jeu de données à une installation de traitement de données.

17. Procédé selon la revendication 15 ou 16, caractérisé par le fait que des images du champ opératoire sont transmises en continu à l'unité de traitement d'images.

18. Procédé selon une des revendications 15 à 17, caractérisé par le fait qu'on produit des images stéréoscopiques du champ opératoire à l'aide de deux caméras vidéo disposées l'une à côté de l'autre.

19. Procédé selon une des revendications 15 à 18, caractérisé par le fait qu'on compare le jeu de données transmis par l'unité de traitement d'images au jeu de données de la représentation théorique dans l'unité de traitement de données.

20. Procédé selon une des revendications 1 à 19, caractérisé par le fait qu'avant l'intervention on mesure la position dans l'espace du champ opératoire dans un système de coordonnées stationnaire.

21. Procédé selon une des revendications 1 à 20, caractérisé par le fait qu'on calcule des facteurs de corrélation pour amener en recouvrement les systèmes de coordonnées et/ou l'échelle de la représentation réelle et de la représentation théorique.

22. Procédé selon la revendication 21, caractérisé par le fait qu'on produit de manière assistée par ordinateur une représentation de la surface d'incision corrigée, amenée en recouvrement avec la représentation réelle.

23. Procédé selon la revendication 20 ou 21, caractérisé par le fait qu'ensuite on injecte la représentation corrigée de la surface d'incision dans la marche des rayons du microscope opératoire.

24. Procédé selon la revendication 20 ou 21, caractérisé par le fait qu'ensuite on reproduit la représentation corrigée de la surface d'incision sur un dispositif de reproduction vidéo en la superposant à la représentation réelle.

25. Procédé selon la revendication 20 ou 21, caractérisé par le fait qu'ensuite on projette la représentation corrigée de la surface d'incision sur la zone du champ opératoire.

26. Procédé selon une des revendications 1 à 25, caractérisé par le fait que la surface d'incision projetée est observée par une personne réalisant l'intervention à l'aide d'un dispositif qui produit une image stéréoscopique de la surface d'incision.

27. Procédé selon une des revendications 1 à 26, caractérisé par le fait qu'on mesure en continu ou de manière discontinue la position dans l'espace par rapport au système de coordonnées stationnaire d'au moins un instrument chirurgical utilisé pour l'intervention.

28. Procédé selon la revendication 27, caractérisé par le fait qu'en cas d'écarts de l'instrument par rapport à la surface d'incision, un signal d'alerte acoustique ou optique est activé.

29. Procédé selon la revendication 27 ou 28, caractérisé par le fait que le guidage de l'instrument le long de la surface d'incision est assisté par un système de guidage d'instrument commandé par l'installation de traitement de données.

30. Procédé selon une des revendications 1 à 29, caractérisé par le fait qu'en plus de la surface d'incision, au moins une ligne de référence pour les parties de tissu vulnérables, telles que des vaisseaux, des nerfs ou similaires et/ou au moins une ligne de résection sont enregistrées sous forme de jeu de données et sont reproduites en superposition sur la représentation réelle ou sont projetées sur le champ opératoire.

31. Dispositif pour la planification et le contrôle d'une intervention chirurgicale, en particulier pour la mise en oeuvre du procédé selon une des revendications 1 à 30, comprenant une installation de traitement de données pour produire et mémoriser au moins une représentation d'un champ opératoire à partir de données structurelles préalablement déterminées, caractérisé par le fait qu'une représentation d'une surface d'incision qui s'étend à travers l'image tridmensionnelle du champ opératoire peut être produite et mémorisée dans l'installation de traitement de données, représentation selon laquelle des tissus sont séparés dans le champ opératoire et par le fait qu'il est prévu un dispositif de reproduction vidéo pour projeter une la représentation théorique ainsi produite sur un cliché du champ opératoire.

32. Dispositif selon la revendication 31, caractérisé par le fait que le dispositif de reproduction vidéo est agencé pour projeter la représentation théorique sur le champ opératoire pendant l'opération.

33. Dispositif selon la revendication 31, caractérisé par le fait qu'on superpose électroniquement la représentation théorique à une représentation réelle du champ opératoire produite de manière intra-opératoire.

34. Dispositif selon une des revendications 31 à 33, caractérisé par un dispositif pour la mesure du champ opératoire.

35. Dispositif selon une des revendications 31 à 34, caractérisé par le fait que le dispositif de reproduction d'image produit deux demi images qui sont superposées l'une à l'autre en une séquence rapide.

36. Dispositif selon la revendication 35, caractérisé par une unité de représentation vidéo stéréoscopique pour la personne réalisant l'intervention qui est commandée de manière synchrone avec le dispositif de reproduction d'image.

37. Dispositif selon une des revendications 31 à 36, caractérisé par un laser pour projeter la représentation théorique et/ou la représentation de la surface d'incision.

38. Dispositif selon une des revendications 31 à 37, caractérisé par deux tubes de projection vidéo pour projeter la représentation théorique et/ou la représentation de la surface d'incision.

39. Dispositif selon une des revendications 31 à 38, caractérisé par un dispositif de déviation commandé par l'installation de traitement de données pour dévier la représentation projetée.

40. Dispositif selon la revendication 31, caractérisé par au moins un appareil de prise de vues pour produire une représentation réelle continue du champ opératoire qui peut être amenée en recouvrement avec au moins une représentation théorique du champ opératoire produite par l'installation de traitement de données à partir de données structurelles préalablement déterminées et mémorisées en même temps qu'au moins une surface d'incision planifiée, ainsi que par au moins un dispositif de reproduction vidéo pour la représentation de la surface d'incision et/ou de la représentation théorique superposée à l'image réelle.

41. Dispositif selon une des revendications 31 à 40, caractérisé par un dispositif pour lire des coordonnées d'au moins une surface d'incision représentée en superposition avec l'image théorique.

42. Dispositif selon la revendication 40 ou 41, caractérisé par au moins deux dispositifs de prise de vues pour enregistrer une image réelle stéréoscopique du champ opératoire.

43. Dispositif selon une des revendications 40 à 42, caractérisé par une unité de traitement d'image connectée à l'appareil de prise de vues.

44. Dispositif selon une des revendications 40 à 43, caractérisé par le fait que le dispositif de reproduction vidéo est disposé dans le champ de vision d'une personne réalisant l'intervention.

45. Dispositif selon une des revendications 40 à 44, caractérisé par le fait que le dispositif de reproduction vidéo est un moniteur.

46. Dispositif selon une des revendications 31 à 45, caractérisé par un microscope d'opération avec des moyens pour injecter la représentation théorique et/ou la surface d'incision dans la marche des rayons du microscope.

47. Dispositif selon une des revendications 31 à 46, caractérisé par au moins un instrument chirurgical couple à un dispositif de mesure pour mesurer ses coordonnées.

48. Dispositif selon une des revendications 31 à 47, caractérisé par au moins un instrument chirurgical couplé à un moyen de guidage d'instrument commandé par l'installation de traitement de données.
